# EUROPÄISCHE PATENTANMELDUNG

(11) **EP 1 036 550 A2**
(43) Veröffentlichungstag der Anmeldung: **20.09.2000**
(21) Anmeldenummer: 00105568.0
(22) Anmeldetag: 16.03.2000
(51) Int. Cl.: A61F 2/06

(54) **Blutgefässstützvorrichtung**

(30) Priorität: 16.03.1999 DE 29904817 U
(71) Anmelder: amg Handelsgesellschaft für angewandte Medizin- und Gesundheitstechnik mbH, 46348 Raesfeld-Erle (DE)
(72) Erfinder: Schober, Gerhard, 85049 Ingolstadt (DE)
(74) Vertreter: Becker Kurig Straus

(57) **Zusammenfassung**

Offenbart wird eine Blutgefäßstützvorrichtung, die ein längliches Metalldrahtgeflecht (2,4) aufweist, das in radialer Richtung spreizbar bzw. dehnbar ist, wobei das Metalldrahtgeflecht (2,4) an mindestens einem axialen Ende mehrere einzelne vorspringende Elemente (10, 12; 10) aufweist.

## Beschreibung

Die vorliegende Erfindung betrifft eine Blutgefäßstützvorrichtung zur Einführung in und Stützung bestimmter Gefäßabschnitte des menschlichen Blutkreislaufs. Derartige Blutgefäßstützvorrichtungen sind auch unter dem Namen Stent in der Medizin bekannt. Die Blutgefäßstützvorrichtungen werden beispielsweise mittels eines Ballonkatheters in den verengten und abzustützenden Gefäßabschnitt eingeführt, an der gewünschten Stelle durch den Ballonkatheter gedehnt und verbleiben dann in dieser Position auch nach Entfernung des Ballonkatheters.

Herkömmliche Blutgefäßstützvorrichtungen sind nur unzureichend für die Abstützung von Gefäßbifurkationen geeignet. Es ist schwierig, die Gefäßstütze so zu positionieren, daß sie wirksam eine Bifurkation abdeckt. Ferner wurde festgestellt, daß die Restenoserate insbesondere an den Enden der Gefäßstütze trotz verbessertem Design und bestimmter Oberflächenbehandlung noch erheblich ist.

Aufgabe der vorliegenden Erfindung besteht daher darin, eine Blutgefäßstützvorrichtung derart weiterzuentwickeln, daß sie auch geeignet ist, Verengungen bei Gefäßabzweigungen, sogenannte Bifurkationsstenosen wirksam zu behandeln. Ferner ist Aufgabe der vorliegenden Erfindung die Angabe einer Blutgefäßstützvorrichtung, die geeignet ist, die Restenoserate an den Enden der Gefäßstütze wirksam zu vermindern.

Gemäß einem ersten Aspekt der vorliegenden Erfindung wird die Aufgabe dadurch gelöst, daß das Metalldrahtgeflecht an mindestens einem axialen Ende mehrere einzelne vorspringende Elemente aufweist. Damit endet das Metalldrahtgeflecht nicht abrupt, sondern es findet ein fließender Übergang zwischen dem Ende der Gefäßstütze und der Gefäßwand bzw. Gefäßintima statt. Die vorspringenden Elemente verlängern die Blutgefäßstütze und führen dadurch eine Reduktion der Stenosen an den Enden der Gefäßstütze herbei.

Bevorzugt sind die Elemente stäbchenförmig ausgebildet und springen in axialer Richtung vor. Auf diese Weise wird eine möglichst gleichmäßige Verlängerung des Metalldrahtgeflechts erzielt.

Bevorzugt sind die Elemente zulaufend ausgebildet. Damit wird der Charakter des fließenden Übergangs verstärkt, so daß die Restenoserate weiter sinkt.

Die Elemente weisen bevorzugt an ihrem äußersten Ende eine Verdickung auf. Damit ist bei bestimmten Anwendungen in Gefäßbifurkationen eine wirksame Befestigung der Blutgefäßstützvorrichtung an der Gefäßwand bzw. an einer weiteren Blutgefäßstützvorrichtung möglich.

Entlang des Umfangs des Metalldrahtgeflechts sind bevorzugt 3-10 Elemente verteilt angeordnet. Diese Anzahl der Elemente hat sich in der Praxis als bevorzugt im Sinne einer Verminderung der Restenoserate herausgestellt.

Bevorzugt sind an beiden Enden des Metalldrahtgeflechtes vorspringende Elemente angeordnet. Für diese gelten analog auch die vorstehend genannten bevorzugten Ausführungsformen. Bei beidseitiger Anordnung der Elemente läßt sich auch beidseitig die Restenoserate wirksam vermindern.

Gemäß einem zweiten Aspekt der vorliegenden Erfindung wird eine Blutgefäßstützvorrichtung angegeben, die dadurch gekennzeichnet ist, daß das Metalldrahtgeflecht mindestens zwei voneinander axial getrennte Geflechtabschnitte aufweist, die durch einzelne Brückenelemente verbunden sind. Diese Blutgefäßstützvorrichtung leistet eine besonders wirksame Abstützung der Gefäßwand bei Bifurkationsstenosen. Die Blutgefäßstütze wird derart eingebracht, daß die beiden Geflechtsabschnitte an den unverzweigten Gefäßwandabschnitten vor und hinter der Abzweigung bzw. Bifurkation zu liegen kommen. Der Bifurkationsbereich ist aufgrund der einzelnen wenigen Brückenelemente weitgehend freigelassen und kann daher einer gesonderten separaten Abstützung unterzogen werden. Diese Blutgefäßstützvorrichtung ist daher besonders geeignet für Stützaufgaben in Gefäßbereichen, in denen ein mittlerer Abschnitt zu überbrücken ist.

Bevorzugt sind die Brückenelemente gekrümmt. Dadurch hat sich in der Praxis eine anwendungstechnisch bevorzugte Überbrückung der Bifurkationsbereiche herausgestellt. Die Brückenelemente sind bevorzugt bogenförmig ausgebildet, was ihre Wirkung weiter erhöht. Besonders bevorzugt sind die Brückenelemente S-förmig ausgebildet.

In einer dritten Ausführungsform der Erfindung wird eine Blutgefäßstützanordnung angegeben, die aus einer Blutgefäßstützvorrichtung gemäß dem ersten Aspekt der Erfindung und einer Blutgefäßstützvorrichtung gemäß dem zweiten Aspekt der vorliegenden Erfindung gebildet ist. Diese Blutgefäßstützanordnung kann insbesondere bei Gefäßbifurkationen eine wirksame Abstützung beider Bifurkationsbereiche ermöglichen. Zunächst kann eine Blutgefäßstützvorrichtung gemäß einem ersten Aspekt der Erfindung mit vorspringenden Elementen in einen Gefäßseitenast eingebracht werden. Durch bestimmte Manipulation können die vorspringenden Elemente am Ostium eingreifen und die Blutgefäßstütze fixieren. Sodann wird die Blutgefäßstützvorrichtung gemäß dem zweiten Aspekt der Erfindung mit einzelnen Brückenelementen im Hauptgefäß so plaziert, daß die Brückenelemente den bereits mit einer anderen Blutgefäßstütze versehenen Seitenast überbrücken und auf diese Weise nach Spreizung beide Blutgefäßstützen wirksam fixieren. Die beiden Blutgefäßstützvorrichtungen wirken somit als Einheit in der Blutgefäßstützanordnung zusammen.

Bevorzugt ist die Blutgefäßstützanordnung so ausgebildet, daß die beiden Blutgefäßstützvorrichtungen dazu bestimmt sind, ineinanderzugreifen bzw. verschachtelt zu sein.

Mit der Kombination der beiden Blutgefäßstützvorrichtungen gemäß dem ersten und dem zweiten Aspekt der Erfindung ist gewährleistet, daß die Blutgefäßstütze im Seitenast nicht mehr in das Hauptgefäß zurückrutschen kann. Ferner wird ein physiologischer Abgang des Seitenastes gewährleistet. Schließlich werden auf diese Art und Weise bislang mit herkömmlichen Gefäßstützen unabgedeckte Bereiche der Gefäßbifurkation bzw. des Ostiums vollständig abgedeckt.

Weitere Anwendungsmöglichkeiten der vorliegenden Erfindung ergeben sich aus der nachfolgenden Beschreibung von drei Ausführungsbeispielen in Verbindung mit der Zeichnung.
Fig. 1 zeigt einen Querschnitt durch eine Ausführungsform einer Blutgefäßstützvorrichtung gemäß einem ersten Aspekt der Erfindung.
Fig. 2 zeigt einen Querschnitt durch eine weitere Ausführungsform einer Blutgefäßstützvorrichtung gemäß einem ersten Aspekt der Erfindung.
Fig. 3 zeigt einen Querschnitt durch eine Ausführungsform einer Blutgefäßstützvorrichtung gemäß einem zweiten Aspekt der Erfindung.

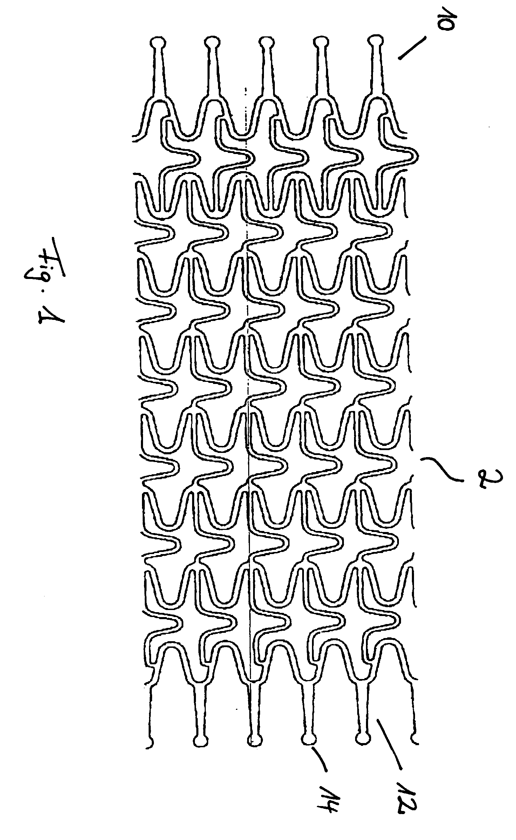

Die in Fig. 1 im Querschnitt gezeigte Blutgefäßstützvorrichtung weist ein im wesentlichen rohrkreiszylindrisches längliches Metalldrahtgeflecht 2 auf, das so ausgebildet ist, daß es in axialer Richtung, beispielsweise durch Dehnung von innen, spreizbar bzw. dehnbar ist und in der gedehnten Position verharrt. An seinem vorderen axialen Ende besitzt das Metalldrahtgeflecht 2 mehrere axial vorspringende stäbchenförmige Elemente 10, die in der zylindrischen Ebene des Metalldrahtgeflechts 2 bleiben. An seinem hinteren Ende besitzt das Metalldrahtgeflecht 2 mehrere ebenfalls stäbchenförmige axial vorspringende Elemente 12. Die Elemente 10 und die Elemente 12 sind leicht zulaufend ausgebildet, beispielsweise konisch und haben an ihrem äußersten Ende eine kleine etwa kugelförmige Verdickung 14. Es sind längs des Umfangs etwa 5-6 Elemente 10 und 5-6 Elemente 12 vorgesehen. Die Implantation dieser Gefäßstütze erfolgt in herkömmlicher Weise mit Ballonkatheter in alle Stenosen, die mit Gefäßstützen zu versorgen sind. Durch wissenschaftliche Studien wurde bewiesen, daß die Restenoserate sich im Laufe der Zeit gewandelt hat. So entstanden früher die Mehrzahl der Restenosen direkt in der Gefäßstütze. Durch verbesserte Gefäßstützende-signs und optimale Oberflächenbehandlung wurden die Stenosen an die Enden der Gefäßstütze verlagert. Durch Verlängerung des Metalldrahtgeflechts aufgrund der Elemente 10, 12 tritt nun kein abruptes Ende des Metallgitters an der Gefäßintima auf, sondern es ergibt sich ein fließender Übergang zwischen dem Ende des Metalldrahtgeflechts und der reinen Gefäßintima. Dadurch wird eine Reduktion der Stenosen an den Geflechtenden herbeigeführt.

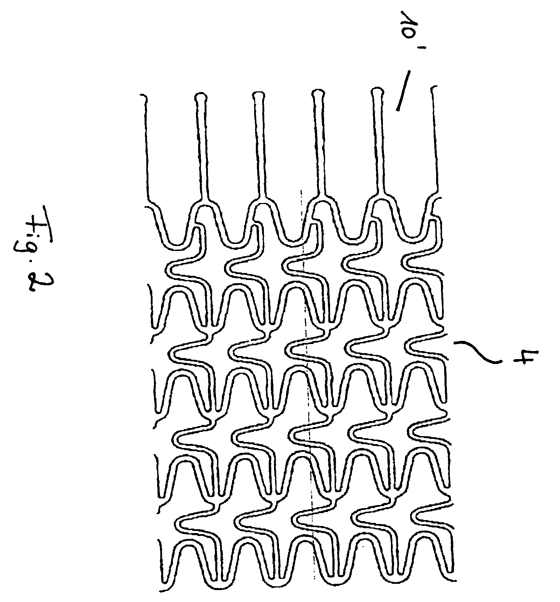

Fig. 2 zeigt eine weitere Ausführungsform einer Gefäßstützvorrichtung gemäß einem ersten Aspekt der Erfindung. Im Gegensatz zu der Ausführungsform von Fig. 1 fehlen hier die Elemente 12 im hinteren Bereich. Es sind lediglich Elemente 10' im vorderen Bereich vorgesehen. Diese Gefäßstütze eignet sich insbesondere für eine Gefäßabstützung eines Seitenastes im Bereich einer Gefäßbifurkation. Dabei sind die Elemente 10' geeignet, den Bereich des Ostiums abzudecken bzw. darin einzugreifen bzw. die Gefäßstütze dort wirksam zu fixieren. Zu diesem Zweck sind die Elemente 10' im wesentlichen mit gleicher Querschnittsfläche über ihre Länge ausgestattet. Die Gefäßstütze 4 wird in einen Gefäßseitenast eingebracht. Durch gezieltes Manipulieren des Ballonkatheters werden die Elemente am Ostium anmodelliert und damit die Gefäßstütze fixiert.

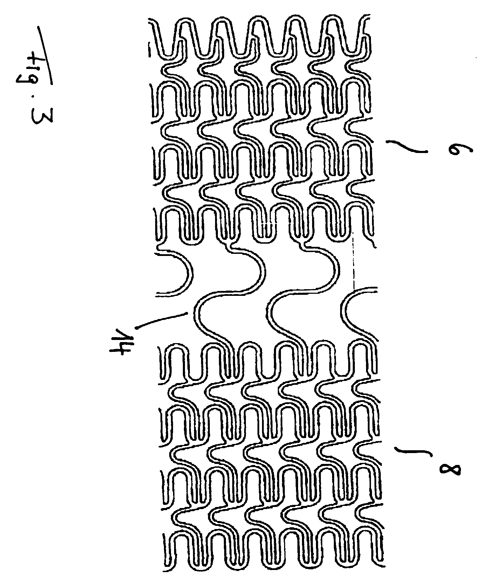

Fig. 3 zeigt eine Ausführungsform einer Gefäßstützvorrichtung gemäß einem zweiten Aspekt der vorliegenden Erfindung. Die Gefäßstützvorrichtung weist zwei voneinander axial getrennte Geflechtabschnitte 6, 8 auf, die in ihrer Ausbildung jeweils den Metalldrahtgeflechten 2, 4 aus Fig. 1, 2 ähneln. Die beiden Geflechtabschnitte 6, 8 sind durch einzelne Brückenelemente 14 verbunden, die in der vorliegenden Ausführungsform S-förmig ausgebildet sind. Die S-förmigen Brückenelemente liegen in der durch die beiden Abschnitte 6, 8 definierten zylindrischen Ebene und stellen einen Brückenbereich zwischen den Geflechtabschnitten 6, 8 dar, der im wesentlichen durchlässig ist. Dadurch ergibt sich insbesondere eine Abstützung von Hauptgefäßen im Bifurkationsbereich. Beispielsweise kann nach Einbringen einer Gefäßstütze nach Fig. 2 eine Gefäßstützvorrichtung nach Fig. 3 in der Mitte im Hauptgefäß so plaziert werden, daß der durch die S-förmigen Elemente definierte Brückenbereich den Seitenastabgang überdeckt. Bei der Expansion der Gefäßstütze werden nun die Elemente 10' der bereits im Seitenast plazierten Gefäßstütze 4 zwischen die Brückenelemente 14 und die Gefäßintima in diesem Bereich gedrückt, so daß eine wirksame Fixierung beider Gefäßstützen möglich ist und zu einer vollständigen Abdeckung des Bifurkationsbereichs führt.

Somit kann durch Verwendung von zwei separaten Gefäßstützen eine normale Standardeinführhilfe verwendet werden, bei der beide Gefäßstützen in der Gefäßbifurkation wieder zu einer Einheit zusammengefügt werden. Dies ist der dritte Aspekt der vorliegenden Erfindung, nämlich die Blutgefäßstützanordnung. Dabei ist die Blutgefäßstütze 4 durch seine Elemente 10' so fixiert, daß sie nicht in das Hauptgefäß zurückrutschen kann. Gleichzeitig wird ein physiologischer Abgang des Seitenastes gewährleistet. Schließlich werden durch geeignete Anmodellierung der Elemente 10' im Ostiumbereich alle Stenosebereiche abgedeckt, die durch Einbringung von zwei herkömmlichen Gefäßstützen frei bleiben würden.

Die vorliegende Erfindung stellt somit einen erheblichen Fortschritt der medizinischen Technik bei der Behandlung, Dehnung und Abstützung von Stenosen des menschlischen Blutkreislaufs dar.

## Patentansprüche

1. Blutgefäßstützvorrichtung, aufweisend ein längliches Metalldrahtgeflecht (2, 4), das in radialer Richtung spreizbar bzw. dehnbar ist, dadurch gekennzeichnet, daß das Metalldrahtgeflecht (2, 4) an mindestens einem axialen Ende mehrere einzelne vorspringende Elemente (10, 12; 10') aufweist.

2. Blutgefäßstützvorrichtung nach Anspruch 1, dadurch gekennzeichnet, daß die Elemente (10, 12; 10') stäbchenförmig ausgebildet sind, in axialer Richtung vorspringen und das Metalldrahtgeflecht (2, 4) verlängern.

3. Blutgefäßstützvorrichtung nach Anspruch 1 oder 2, dadurch gekennzeichnet, daß die Elemente (10, 12) zulaufend ausgebildet sind.

4. Blutgefäßstützvorrichtung nach einem der vorhergehenden Ansprüche, dadurch gekennzeichnet, daß die Elemente (10, 12; 10') an ihrem äußersten Ende eine Verdickung (14) ausweisen.

5. Blutgefäßstützvorrichtung nach einem der vorhergehenden Ansprüche, dadurch gekennzeichnet, daß entlang des Umfangs des Metalldrahtgeflechts (2, 4) 8-15 Elemente (10, 12; 10') verteilt angeordnet sind.

6. Blutgefäßstützvorrichtung nach einem der vorhergehenden Ansprüche, dadurch gekennzeichnet, daß an beiden Enden des Metalldrahtgeflechtes vorspringende Elemente (10, 12) angeordnet sind.

7. Blutgefäßstützvorrichtung, aufweisend ein längliches Metalldrahtgeflecht (6, 8), das in radialer Richtung spreizbar bzw. dehnbar ist, dadurch gekennzeichnet, daß das Metalldrahtgeflecht mindestens zwei von einander axial getrennte Geflechtabschnitte (6, 8) aufweist, die durch einzelne Brückenelemente (14) verbunden sind.

8. Blutgefäßstützvorrichtung nach Anspruch 7, dadurch gekennzeichnet, daß die Brückenelemente (14) gekrümmt sind.

9. Blutgefäßstützvorrichtung nach Anspruch 8, dadurch gekennzeichnet, daß die Brückenelemente (14) bogenförmig ausgebildet sind.

10. Blutgefäßstützvorrichtung nach Anspruch 9, dadurch gekennzeichnet, daß die Brückenelemente (14) S-förmig ausgebildet sind.

11. Blutgefäßstützanordnung, gebildet aus einer Blutgefäßstützvorrichtung gemäß einem der Ansprüche 1-6 und einer Blutgefäßstützvorrichtung gemäß einem der Ansprüche 7-10.

12. Blutgefäßstützanordnung nach Anspruch 11, dadurch gekennzeichnet, daß die beiden Blutgefäßstützvorrichtungen (2, 4; 6, 8) dazu bestimmt sind, ineinanderzugreifen bzw. verschachtelt zu sein.
